# EUROPEAN PATENT APPLICATION

(11) **EP 2 116 515 A1**
(43) Date of publication of application: **11.11.2009**
(21) Application number: 08700637.5
(22) Date of filing: 11.01.2008
(51) Int. Cl.: C01G 37/033, C01G 49/00, C07C 55/07, C07C 51/41, C22B 34/32

(54) **A METHOD OF PREPARING CHROMIC OXIDE AND FERROUS OXALATE FROM CARBON FERROCHROME**

(30) Priority: 26.01.2007 CN 200710034327
(71) Applicant: Hunan Joyfly Technology Development Co., Ltd., Changsha Hunan 410-006 (CN)
(72) Inventor: HU, Guohua, Hunan 410000 (CN)
(74) Representative: Hryszkiewicz, Danuta
(86) International application number: PCT/CN2008/000084
(87) International publication number: WO 2008/092363

(57) **Abstract**

The present invention disclosed a method of preparing chromic oxide and ferrous oxalate from carbon ferrochrome. The carbon ferrochrome which is cheap and easily obtainable is used as starting material. The carbon ferrochrome is dissolved in sulfuric acid, then oxalic acid is added to the solution to separate chrome and iron, and then ferrous oxalate powder with total impurities content less than 1% and chromic oxide with iron content less than 0.5% are obtained. The technical conditions are controlled as following: sulfuric acid of 20%-60% is used to dissolve carbon ferrochrome, the dissolving temperature is 80 -150°C, the amount of oxalic acid added is 105% -120% of the theoretical amount to precipitate iron into ferrous oxalate, precipitation time of iron is 20 min - 1 h, and precipitation pH of iron is 2.0 - 4.0.

## Description

This invention relates to a method of preparing chromic oxide and ferrous oxalate, and more particularly to a method of preparing chromic oxide and ferrous oxalate from carbon ferrochrome by a wet chemical processing.

Chromic oxide, sodium dichromate (Na₂Cr₂O₇·2H₂O), chromic anhydride, and basic chromium sulfate are four major chromic salts. Almost all commercial chromic oxide is prepared from sodium dichromate directly or indirectly, and the yield is almost equal to 20% of the sodium dichromate consumption. In the world, the total production capacity of chromic oxide is around 100,000 tons per year and has been increasing year by year. Basically, chromic oxide is used in the following four aspects: raw materials for preparing chrome or advanced non-ferrous chrome alloy, refractory materials, pigments, and abrasives. Recently, some novel chromic oxides have been developed in foreign countries. For example, a kind of chromic oxide specifically used for melt blowing has been disclosed by many literatures and patents. The chromic oxide was sprayed by plasma technology on a target (a metal or ceramic) and formed a protective film with strong adhesion, so the obtained target had excellent performances in wear resistance, temperature resistance, and corrosion resistance. Furthermore, low water absorption of chromic oxide and high void fraction of chromic oxide were also reported. Additionally, chromic oxide can be used as catalyst or carrier for preparing composite oxide, or as materials for preparing chrome-containing carbide, chrome-containing boride, chrome-containing nitride, and chrome-containing silicide.

As an organic chemical product and mainly functioning as chemical reagents, ferrous oxalate is further used in paints, dyes, ceramic glaze, coloring optical glass, coloring glassware, preparing photosensitive materials, and so on.

Conventional industrial methods of preparing chromic oxide include: 1). chromium anhydride pyrolysis method, 2). potassium dichromate-sulfur reduction roasting method, 3). sodium chromate-sulfur wet reduction method, and 4). sodium chromate-sodium sulfide wet reduction method. Currently, in China, in order to obtain chromic oxide, sodium dichromate is acidized with sulfuric acid to yield chromium anhydride and then to roast resultant chromium anhydride under high temperature. However, the process requires high production cost, and produces a large amount of toxic wastewater and chromium-containing gas, which cause serious pollution and damage against eco-environment and humans. For methods with sulfur or sodium sulfide as reducing agents, although the raw materials is easily obtainable, the production period is long, a large amount of sulfur dioxide-containing gas and sulfur-containing wastes are produced, and by-products are difficult to separate, all of which lead to difficulty in obtaining high quality products.

Carbon ferrochrome is a high-grade chrome-containing material. It is inexpensive, easily obtainable, and exists widely in nature. If carbon ferrochrome can be used as raw material to produce chromic oxide, the pollution caused by hexavalent chromium during production may be completely avoided. However, the separation of chrome and iron from carbon ferrochrome is a major problem. Due to the similarity of chromium ion and iron ion, conventional wet methods such as hydroxide precipitate method cannot settle this problem. Some studies try to separate chromium ion and iron ion by chlorinating carbon ferrochrome. The method aims at roasting carbon ferrochrome at high temperature to yield FeCl₃ and Cryl₃, and then separating chrome and iron ion according to boiling spread by condensation under different temperature. However, the method is demanding on the equipment, and also causes safety and environmental problems due to use of chlorine.

Accordingly, in view of the above-described problems, it is one objective of the invention to provide a method of preparing chromic oxide and ferrous oxalate from carbon ferrochrome that is simple and economic, has high metal recovery rate, and causes no pollution.

To achieve the above objectives, in accordance with one embodiment of the invention, there is provided a method of preparing chromic oxide and ferrous oxalate from carbon ferrochrome that is simple and economic, has high metal recovery rate, and causes no pollution. The technical solution of the method comprises dissolving carbon ferrochrome with sulfuric acid to yield a solution comprising trivalent chromium ion and ferrous ion, and adding oxalic acid to the solution. Since oxalic acid reacts with ferrous ion to yield a precipitate of ferrous oxalate, while no precipitate is produced between oxalic acid and trivalent chromium ion, thereby chromic oxide is separated from carbon ferrochrome, and iron is converted to ferrous oxalate.

Specifically, the method comprises the steps of:
a) breaking carbon ferrochrome and adding into a reactor, adding sulfuric acid in batches at a temperature for dissolving, and filtering to yield an acid solution;
b) adding ammonia to the acid solution for adjusting the pH value, adding oxalic acid, adding ammonia for maintaining the pH value, stirring, filtering, and drying in an oven to yield ferrous oxalate; and
c) adding ammonia to a filtrate of b) for adjusting the pH value to yield chromium hydroxide, filtering, drying, and roasting the chromium hydroxide in a muffle furnace to yield chromic oxide.

In a class of this embodiment, in the step a), the carbon ferrochrome is broken into pieces with size of between 1 and 100 µm.

In a class of this embodiment, in the step a), the temperature is between 80 and 150°C, and a dissolving time between 0.5 and 2 h.

In a class of this embodiment, in the step a), the carbon ferrochrome comprises iron and 30 - 80% chrome.

In a class of this embodiment, in the step a), the sulfuric acid has a concentration of 20 - 60%, and is added to the reactor in 2 - 5 batches or dropwise continuously.

In a class of this embodiment, in the step b), the ammonia is added dropwise and the pH value is between 2.0 and 5.0.

In a class of this embodiment, in the step b), an adding amount of the oxalic acid is 105 - 120 % of the theoretical amount of that for precipitating ferrous ion into ferrous oxalate, and the oxalic acid is added in the form of solid.

In a class of this embodiment, in the step c), the ammonia is added dropwise slowly and the pH value is between 7.0 and 9.5.

In a class of this embodiment, in the step c), the chromium hydroxide is roasted in the muffle furnace, a roasting temperature between 500 and 900°C, and a roasting time between 2 and 5 h.

Advantages of the invention are summarized below:

1. as raw materials carbon ferrochrome is easily obtainable, and the invention solves the technical problems of separation chrome and iron from carbon ferrochrome, not only chromic oxide is produced, but also high value-added ferrous oxalate is obtained, both of which have high quality;

2. no hexavalent chromium, gas containing sulfuric dioxide, and waste containing sulfur are produced during production, and no pollutants discharged, so the invention controls pollution from the source, causes no damage to eco-environment and humans;

3. the process of the invention is simple, production period is short, equipment has high efficiency, metal recovery rate is high, and production cost is low, so the invention is suitable for mass production.

The invention is described hereinbelow with reference to accompanying drawings, in which:

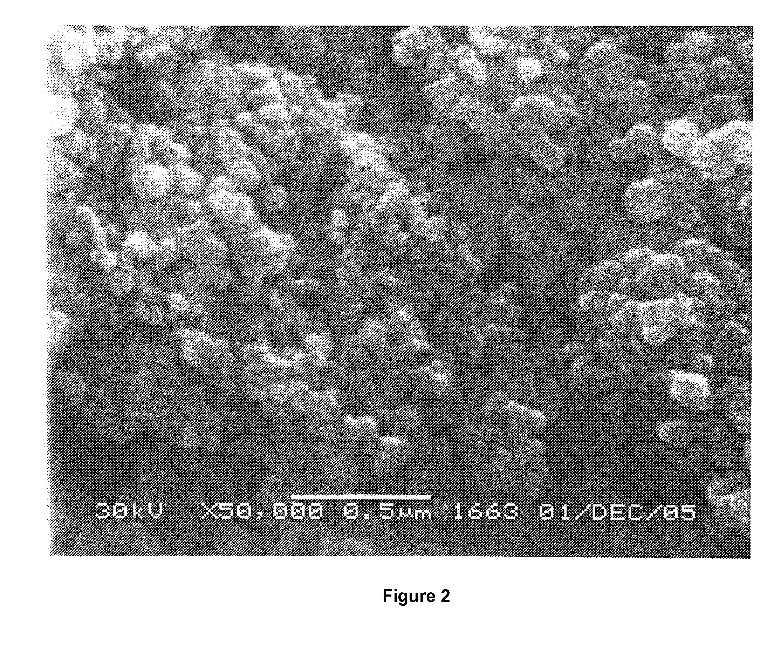

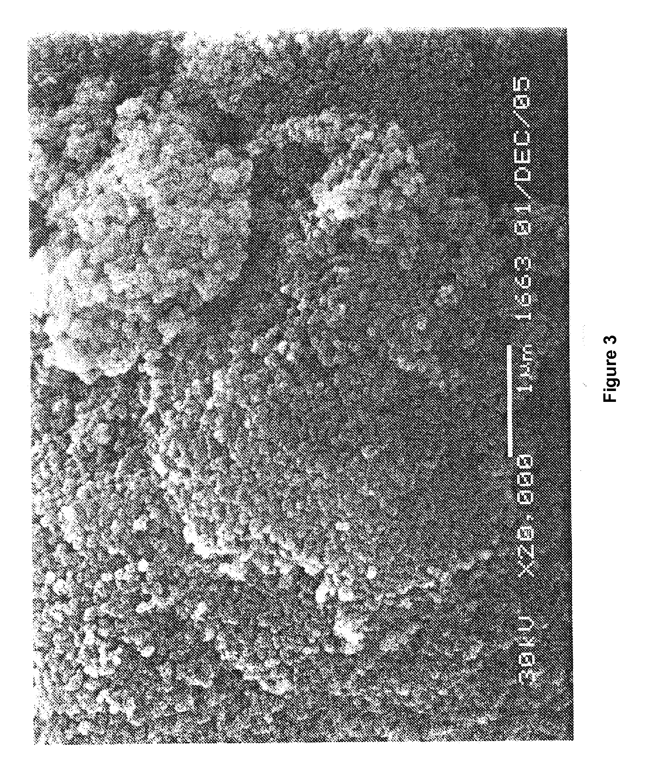

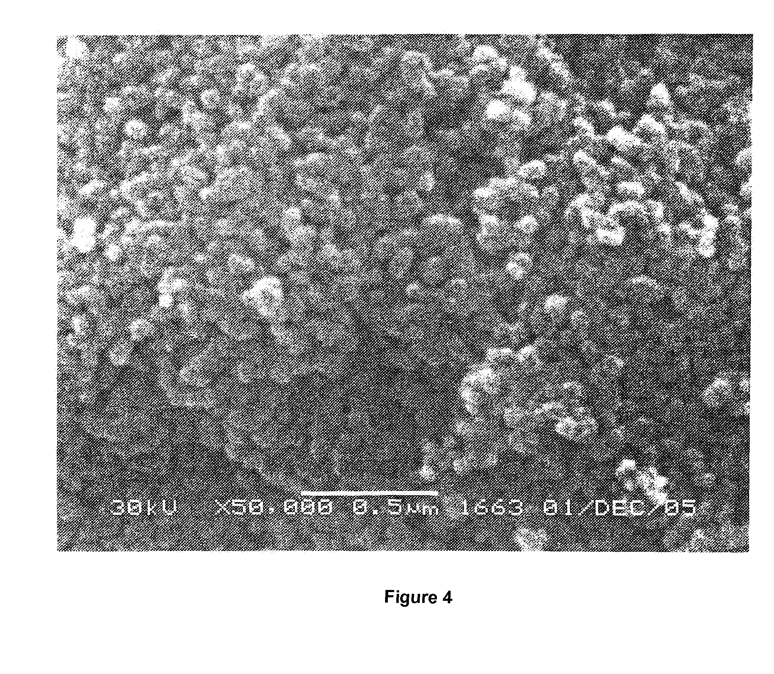

FIGS. 1-4 are SEM images of chromic oxide prepared according to a method of one embodiment of the invention;

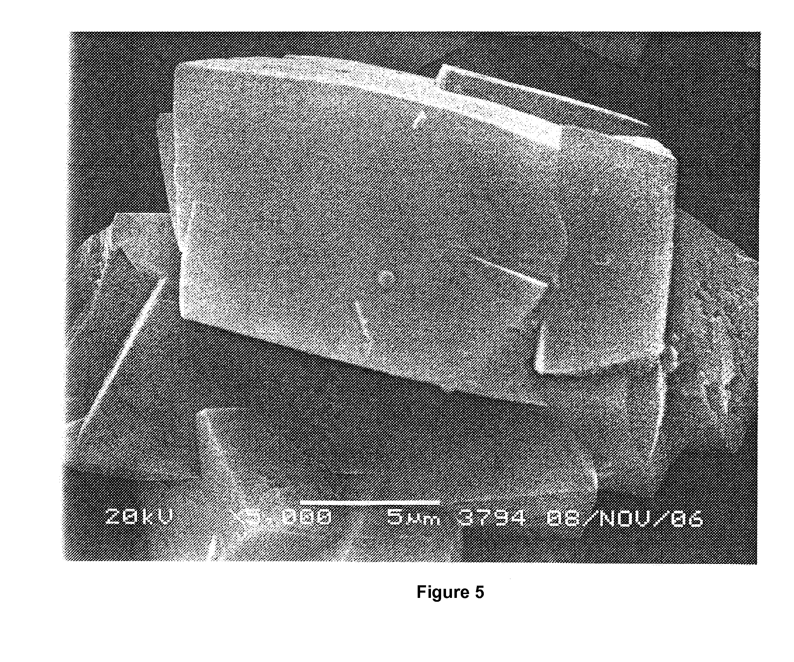

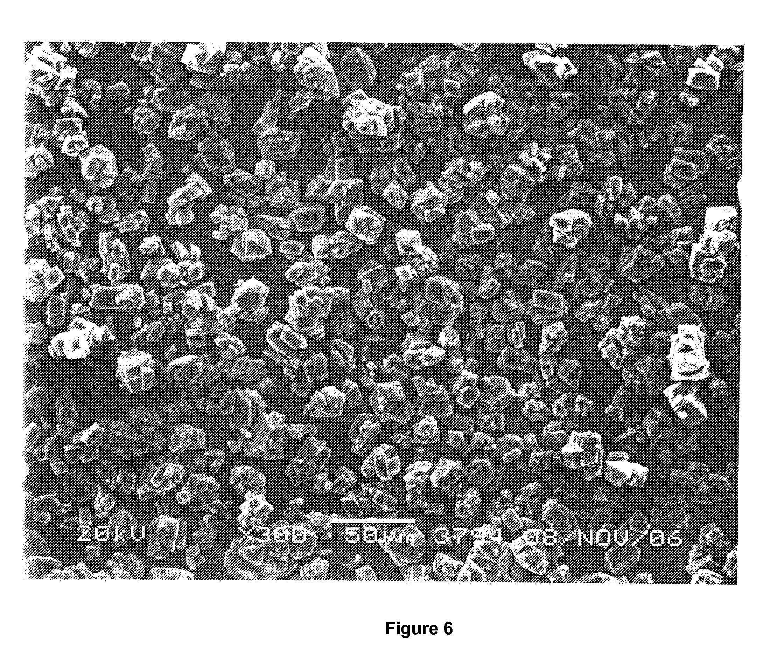

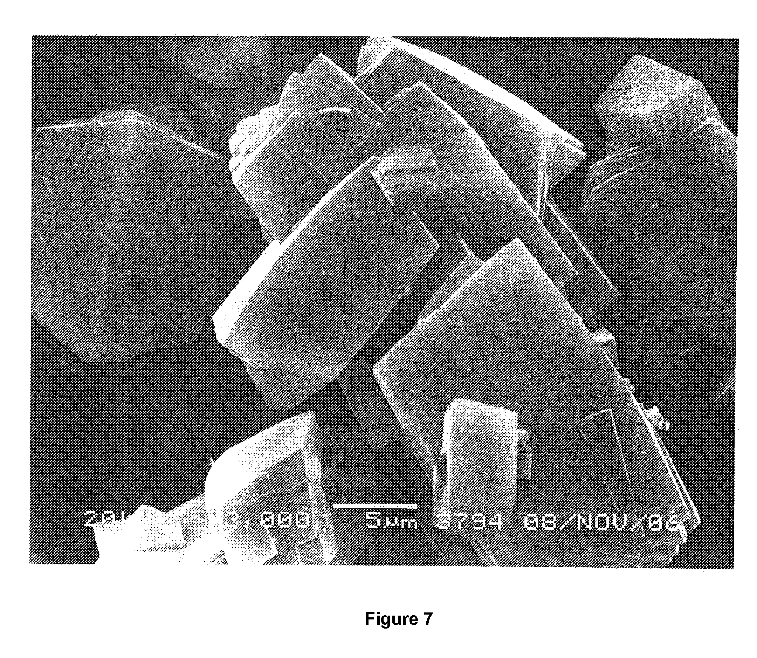

FIGS. 5-8 are SEM images of ferrous oxalate prepared according to a method of one embodiment of the invention;

FIG. 9 shows an XRD pattern of chromic oxide prepared according to a method of one embodiment of the invention; and

FIG. 10 shows an XRD pattern of ferrous oxalate prepared according to a method of one embodiment of the invention.

For further illustrating the invention, experiments detailing the preparation of chromic oxide and ferrous oxalate from carbon ferrochrome are described below. It should be noted that the following examples are intended to describe and not to limit the invention.

Example 1

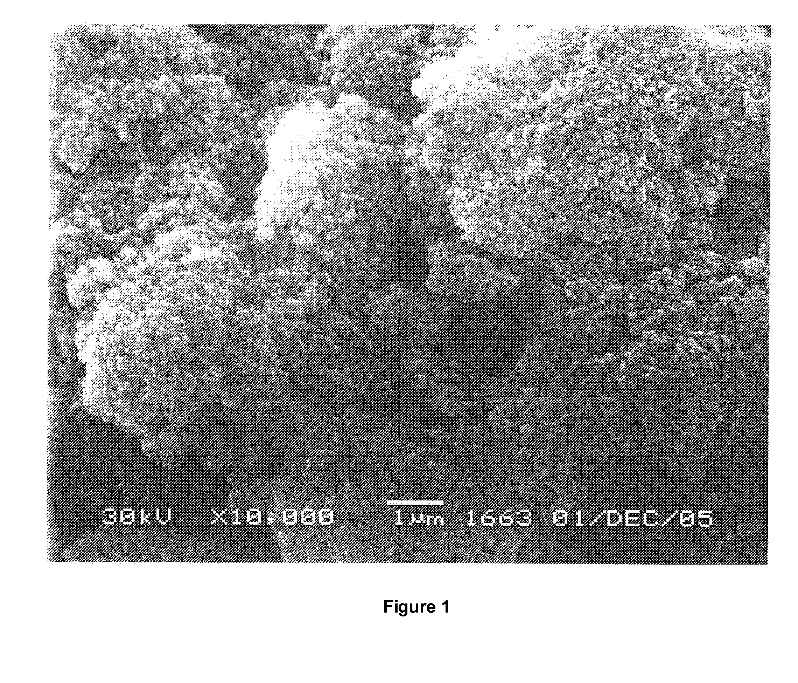

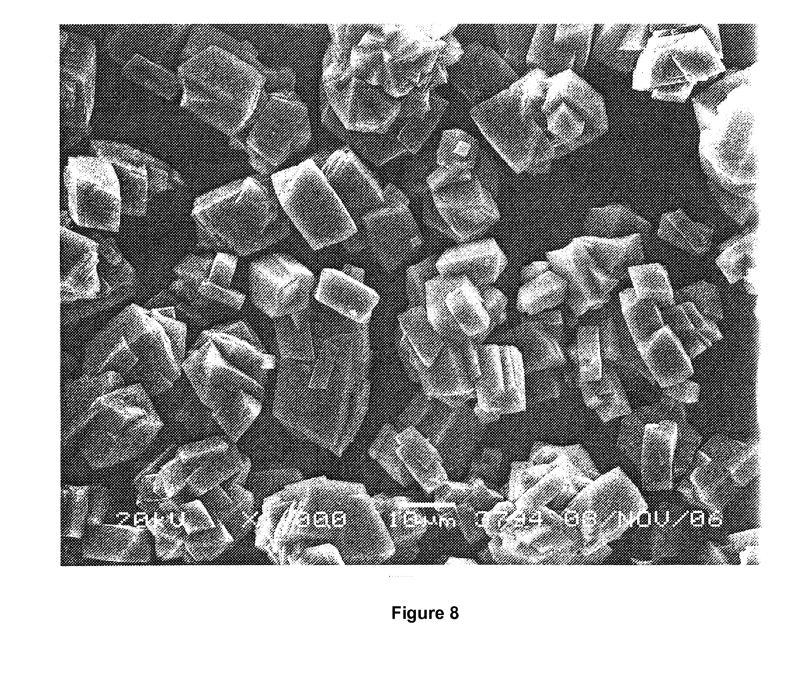

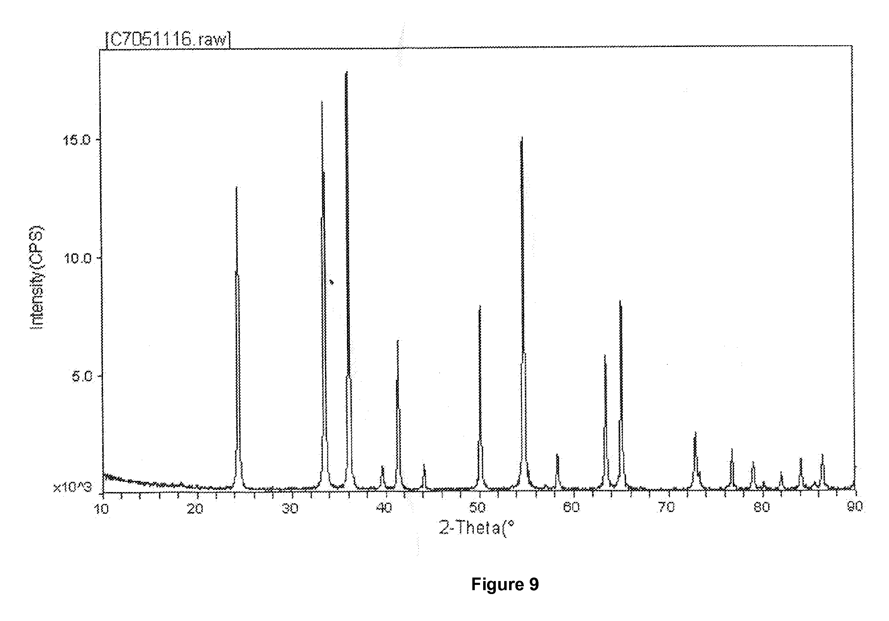

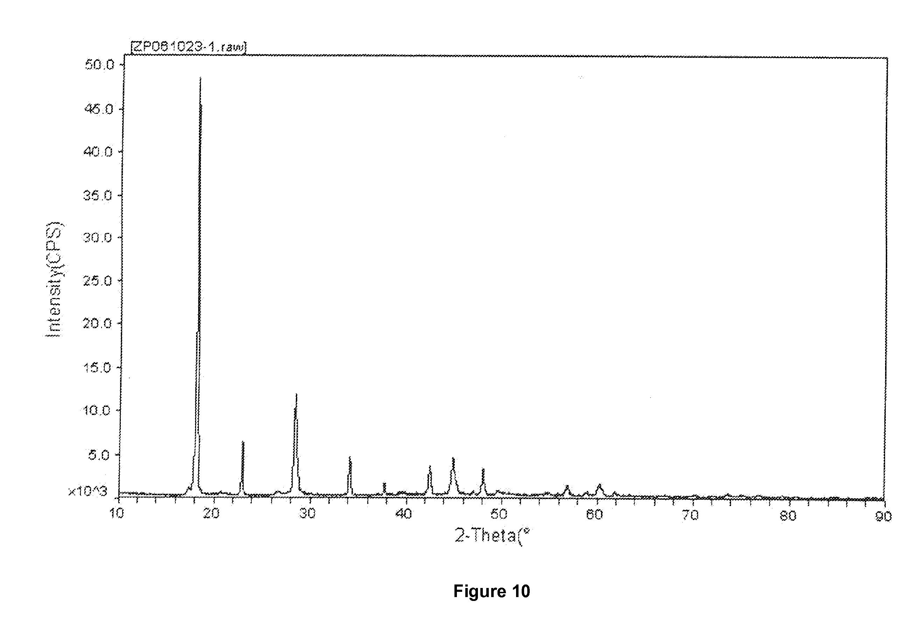

To a glass container, 30 g of broken carbon ferrochrome powder was added. 50 mL of concentrated sulfuric acid was collected and diluted with 50 mL of tap water. The resultant mixture was added to the glass container in four batches with stirring of 300 r/m, heated by an electric cooker for an hour, and filtered to yield an acid solution. The acid solution was diluted to 500 mL, and ammonia was added dropwise with stirring of 300 r/m to adjust the pH value to 3.5. 22 g of oxalic acid was added to the solution, and ammonia was further added to maintain the pH value at 3.5. The mixture was allowed for reaction for half an hour and filtered to yield ferrous oxalate and a chromium sulfate solution. The ferrous oxalate was dried at 120°C for 12 h in an oven to yield 25.4 g of ferrous oxalate product, an XRD pattern of which was shown in FIG. 10, and an SEM image of which was shown in FIG. 8. Ammonia was added to the chromium sulfate solution with stirring of 300 r/m to adjust the pH value to 8.0, and chromium hydroxide was precipitated. The chromium hydroxide was dried at 120°C for 24 h in an oven to yield dried chromium hydroxide. The dried chromium hydroxide was ground and roasted at 800°C for 2 h in a muffle furnace to yield 26.5 g of chromic oxide, an XRD pattern of which was shown in FIG. 9, and an SEM image of which was shown in FIG. 1. Analysis has showed: the content of chromium in the ferrous oxalate was 0.003%, and the total recovery rate of iron was 98.6%; the content of iron in the chromic oxide was 0.3%, and the total recovery rate of chromium was 98.3%.

Example 2

To a glass container, 30 g of broken carbon ferrochrome powder was added. 60 mL of concentrated sulfuric acid was collected and diluted with 90 mL of tap water. The resultant mixture was added to the glass container in three batches with stirring of 200 r/m, heated by an electric cooker at 100°C for an hour, and filtered to yield an acid solution. The acid solution was diluted to 1000 mL, and ammonia was added dropwise with stirring of 300 r/m to adjust the pH value to 3.0. 24 g of oxalic acid was added to the solution, and ammonia was further added to maintain the pH value at 3.0. The mixture was allowed for reaction for 20 minutes and filtered to yield ferrous oxalate and a chromium sulfate solution. The ferrous oxalate was dried at 120°C for 12 h in an oven to yield 25.7 g of ferrous oxalate product. Ammonia was added to the chromium sulfate solution with stirring of 200 r/m to adjust the pH value to 9.0, and chromium hydroxide was precipitated. The chromium hydroxide was dried at 120°C for 24 h in an oven to yield dried chromium hydroxide. The dried chromium hydroxide was ground and roasted at 600°C for 4 h in a muffle furnace to give 25.8 g of chromic oxide. Analysis has showed: the content of chromium in the ferrous oxalate was 0.002%, and the total recovery rate of iron was 98.8%; the content of iron in the chromic oxide was 0.21 %, and the total recovery rate of chromium was 97.8%.

Example 3

To a glass container, 60 g of broken carbon ferrochrome powder by airflow was added. 80 mL of concentrated sulfuric acid was collected and diluted with 70 mL of tap water. The resultant mixture was added dropwise to the glass container continuously with stirring of 400 r/m, heated by an electric cooker at 80°C for 2 h, and filtered to yield an acid solution. The acid solution was diluted to 1000 mL, and ammonia was added dropwise with stirring of 400 r/m to adjust the pH value to 4.5. 60 g of oxalic acid was added to the solution, and ammonia was further added to maintain the pH value at 4.5. The mixture was allowed for reaction for an hour and filtered to yield ferrous oxalate and a chromium sulfate solution. The ferrous oxalate was dried at 120°C for 12 h in an oven to yield 38.3 g of ferrous oxalate product. Ammonia was added to the chromium sulfate solution with stirring of 400 r/m to adjust the pH value to 9.5, and chromium hydroxide was precipitated. The chromium hydroxide was dried at 120°C for 24 h in an oven to yield dried chromium hydroxide. The dried chromium hydroxide was ground and roasted at 900°C for 2 h in a muffle furnace to yield 18.4 g of chromic oxide. Analysis has showed: the content of chromium in the ferrous oxalate was 0.016%, and the total recovery rate of iron was 98.4%; the content of iron in the chromic oxide was 0.36%, and the total recovery rate of chromium was 92.4%.

Example 4

To a glass container, 60 g of broken carbon ferrochrome powder by airflow was added. 80 mL of concentrated sulfuric acid was collected and diluted with 70 mL of tap water. The resultant mixture was added dropwise to the glass container continuously with stirring of 400 r/m, heated by an electric cooker at 150°C for 0.5 h, and filtered to yield an acid solution. The acid solution was diluted to 1000 mL, and ammonia was added dropwise with stirring of 400 r/m to adjust the pH value to 2.5. 40 g of oxalic acid was added to the solution, and ammonia was further added to maintain the pH value at 2.5. The mixture was allowed for reaction for an hour and filtered to yield ferrous oxalate and chromium sulfate solution. The ferrous oxalate was dried at 120°C for 12 h in an oven to yield ferrous oxalate product. Ammonia was added to the chromium sulfate solution with stirring of 400 r/m to adjust the pH value to 7.0, and chromium hydroxide was precipitated. The chromium hydroxide was dried at 120°C for 24 h in an oven to yield dried chromium hydroxide. The dried chromium hydroxide was ground and roasted at 500°C for 5 h in a muffle furnace to give 48.5 g of chromic oxide. Analysis has showed: the content of chromium in the ferrous oxalate was 0.001 %, and the total recovery rate of iron was 90.2%; the content of iron in the chromic oxide was 0.68%, and the total recovery rate of chromium was 89.4%.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A method of preparing chromic oxide and ferrous oxalate from carbon ferrochrome comprising dissolving carbon ferrochrome with sulfuric acid to yield a solution comprising trivalent chromium ion and ferrous ion, and adding oxalic acid to the solution, oxalic acid reacting with ferrous ion to yield a precipitate of ferrous oxalate, while no precipitate being produced between oxalic acid and trivalent chromium ion, thereby chromic oxide being separated from carbon ferrochrome, and iron being converted to ferrous oxalate.

2. The method of claim 1, comprising the steps of:
a) breaking carbon ferrochrome and adding into a reactor, adding sulfuric acid in batches at a temperature for dissolving, and filtering to yield an acid solution;
b) adding ammonia to the acid solution for adjusting the pH value, adding oxalic acid, adding ammonia for maintaining said pH value, stirring, filtering, and drying to yield ferrous oxalate; and
c) adding ammonia to a filtrate of b) for adjusting the pH value to yield chromium hydroxide, filtering, drying, and roasting the chromium hydroxide to yield chromic oxide.

3. The method of claim 2, wherein in the step a), said carbon ferrochrome is broken into pieces with size of between 1 and 100 µm.

4. The method of claim 2, wherein in the step a), said temperature is between 80 and 150°C, and a dissolving time between 0.5 and 2 h.

5. The method of claim 2, wherein in the step a), said carbon ferrochrome comprises iron and 30 - 80% chrome.

6. The method of claim 2, wherein in the step a), said sulfuric acid has a concentration of 20 - 60%, and is added to said reactor in batches of 2 - 5 or dropwise continuously.

7. The method of claim 2, wherein in the step b), said ammonia is added dropwise and said pH value is between 2.0 and 4.0.

8. The method of claim 2, wherein in the step b), an adding amount of said oxalic acid is 105 - 120 % of the theoretical amount of that for precipitating ferrous ion into ferrous oxalate, and said oxalic acid is added in the form of solid.

9. The method of claim 2, wherein in the step c), said ammonia is added dropwise and said pH value is between 7.0 and 9.5.

10. The method of claim 2, wherein in the step c), a roasting temperature is between 500 and 900°C, and a roasting time between 2 and 5 h.
